# EUROPÄISCHE PATENTSCHRIFT

(11) **EP 3 464 445 B1**
(45) Veröffentlichungstag und Bekanntmachung des Hinweises auf die Patenterteilung: **05.08.2020**
(21) Anmeldenummer: 17731815.1
(22) Anmeldetag: 01.06.2017
(51) Int. Cl.: C08K 5/00, C08K 5/353, C08K 3/10, C08K 5/09, C08K 5/1535, C08K 5/3415

(54) **POLYESTER- UND POLYOLEFINFORMMASSEN MIT BIOAKTIVEN EIGENSCHAFTEN UND DARAUS HERGESTELLTE FORMKÖRPER**
POLYESTER AND POLYOLEFIN MOULDING COMPOUNDS WITH BIOACTIVE PROPERTIES AND MOULDED BODIES PRODUCED THEREFROM
MATIÈRES À MOULER POLYESTER ET POLYOLÉFINIQUES PRÉSENTANT DES PROPRIÉTÉS BIOACTIVES ET CORPS MOULÉS FABRIQUÉS À PARTIR DE CELLES-CI

(30) Priorität: 06.06.2016 DE 102016006745
(43) Veröffentlichungstag der Anmeldung: 10.04.2019
(73) Patentinhaber: Thüringisches Institut für Textil- und Kunststoff-Forschung e.V., 07407 Rudolstadt (DE)
(72) Erfinder: STRUBL, Rüdiger, 07318 Saalfeld (DE); HEINEMANN, Klaus, 07407 Rudolstadt (DE); SCHUBERT, Frank, 07751 Jena (DE); BAUER, Ralf-Uwe, 07407 Rudolstadt (DE); RIEDE, Sabine, 07407 Uhlstädt-Kirchhasel (DE)
(74) Vertreter: Plate, Jürgen
(86) Internationale Anmeldenummer: PCT/EP2017/063333
(87) Internationale Veröffentlichungsnummer: WO 2017/211685

(56) Entgegenhaltungen:
- CN-A- 107 083 618
- DE-A1-102010 051 310
- JP-A- 2001 234 448
- JP-A- 2008 163 503
- JP-A- 2012 010 915
- US-A1- 2010 115 706

## Beschreibung

Die Erfindung betrifft aus der Schmelze verformbare Polyester- und Polyolefin-Formmassen, bei denen durch Einsatz spezieller komplexierter Zinksalzverbindungen eine langfristige bioaktive bzw. biocide, insbesondere antibakterielle Wirkung erreicht wird. Die komplexierten Zinksalzverbindungen bestehen aus einer Kombination eines Zinksalzes mit einer heterozyklischen organischen Verbindung, die als Ligand gegenüber dem Metallkation auftritt und zu einer stöchiometrisch aufgebauten, thermisch stabilen Komplexverbindung führt. Die komplexierten Zinksalzverbindungen liegen molekular vor, sie können direkt mit der Polymerschmelze verarbeitet werden, sie beeinflussen weder die Viskosität der Schmelze noch die physikalischen Eigenschaften der Formkörper, sie zeigen in speziellen wasserfreien Formulierungen keine katalytische Wirkung auf einen möglichen Polymerabbau sowohl in Polyestern als auch in Polyolefinen. Das Zink liegt direkt in ionisch stabilisierter Form in der Formmasse vor.

Diese Formmassen lassen sich durch dem Fachmann bekannte Verfahren zur Herstellung von Formkörpern aus Polymerschmelzen zu verschiedenen Formkörpern, wie Fasern, Garne, Folien, Schäume, Platten, Spritzgusserzeugnisse mit permanenten antibakteriellen Eigenschaften verformen.

Derartige Zinksalz-Organoligand-Komplexe, insbesondere auf der Basis von Carbonsäure-(2-Oxazolin)-Derivaten sind prinzipiell bekannt und kamen bereits zur Modifizierung der Eigenschaften von Polyamiden zum Einsatz (EP 2 640 776). Es musste jedoch festgestellt werden, dass im Gegensatz zu Polyamiden mit ihren typischen Restfeuchtigkeitskonzentrationen von etwa (0,03 - 0,07) Prozent die Verwendung der beschriebenen Zinksalzkomplexe zur Eigenschaftsmodifizierung von Polyester- und Polyolefin-Formmassen einerseits auf Grund der zur Erreichung von antibakteriellen Eigenschaften im Vergleich zu dem Fachmann bekannten Silber- bzw. Silberionen-Konzentrationen verhältnismäßig hohen Zinksalzkonzentrationen und deren katalytischen Wirksamkeit für Polymer-, insbesondere für Polyolefinabbaureaktionen sowie andererseits auf Grund ihres darin enthaltenen relativ hohen Wasseranteils und dem damit verbundenen Hydrolysepotenzial im Falle von Polyestern mit sehr erheblichen Schwierigkeiten z. B. bei Anwendung der Compoundier- und Schmelzspinntechnologie in Folge von dramatischen Polymerdegradationsprozessen verbunden war und die ebenda beschriebenen Komplexe deshalb nicht einsetzbar waren. CN 107 08 36 18 A veröffentlicht am 22-08-2017 offenbart die Anwendung von Zinkoxid als antibakterielles Mittel zur Modifizierung von Polyester in der Schmelze.

Die Aufgabenstellung dieser Erfindung besteht somit darin, mittels Polymerschmelzeverformungsverfahren hergestellte Produkte, insbesondere Formkörper, zur Verfügung zu stellen, die aus modifizierten Polyolefin- und Polyesterschmelzen resultieren, in denen nicht agglomerierende, hochtemperaturbeständige und mittels in-line-Technologie inkorporierte Polymeradditive enthalten sind, die ionisch gebundene Zink-basierte Biozide darstellen und mit denen bereits beim Einsatz von geringen Mengen sehr gute antibakterielle Eigenschaften mit hoher Permanenz, großer Langzeitwirkung, überraschender Effizienz sowie kombiniert damit auch hautpflegende Effekte erzielt werden.

Die bekannten Lösungen besitzen den Nachteil, dass hohe Biozidgehalte beispielsweise im Falle von Nanosilber oder Zinkoxid zugesetzt werden müssen, da zur Erlangung einer nennenswerten bioaktiven Wirkung im Gebrauch beispielsweise der Fasern die wirksamen Spezies der ionischen Formen von Silber bzw. Zink in ausreichenden Konzentrationen erst entstehen müssen. Weitere Nachteile sind häufig Agglomerisationen der Zusatzstoffe, welche die Verarbeitung der Polymeren aus Schmelzen zu Multifilamenten empfindlich stören ebenso wie Partikel auf Basis geträgerter anorganischer Zeolithe.

Die Erfindung löst die Aufgabe dadurch, dass Zinksalze mit geeigneten Komplexbildnern für Zinkionen - sog. Organoliganden - verwendet werden, welche Zinkionen auf molekularer Basis kapseln und außerdem eine hohe thermodynamische Kompatibilität sowohl mit Polyolefin- als auch mit Polyesterschmelzen gewährleisten, unter hohen Verarbeitungstemperaturen thermisch beständig sind und im Verarbeitungsprozedere direkt vorteilhafterweise als Masterbatchformulierung eingesetzt werden können, da nur relativ geringe Zinkionenkonzentrationen für eine effiziente Wirksamkeit benötigt werden und es gelungen ist, eine sogenannte "in-situ-Entwässerung" bei der Herstellung dieser Komplexe zu realisieren, da in wasserfreien Medien sowie unter Verwendung wasserfreier Zinksalze keine derartigen Zinkionen-Organoligand-Komplexe auf der Basis von 2-Oxazolin-Derivaten einfacher aliphatischer oder aromatischer Mono- und Dicarbonsäuren entstehen.

Die vorliegende Erfindung besitzt mithin folgende Vorteile:
- Einfache Synthese wasserarmer biozider Wirkstoffe aus preiswerten Rohstoffe
- Universell prozesstaugliche Polymeradditive für die Schmelzeverarbeitung von Polyolefinen und Polyestern beispielsweise, jedoch nicht ausschließlich zu Fasern, Folien, Spritzgussartikeln, andere diverse Formkörper
- Hohe Thermostabilität bei Temperaturen von über 310°C
- thermodynamisch kompatibel mit Polyolefinen und Polyestern
- Keine Polymerdegradationen während der Verarbeitung und Anwendung
- Keine Partikelbildung in Folge von Agglomerationen in den Polymerschmelzen
- In-line-Anwendung der Komplexe im Verarbeitungsprozess
- Adaption an bestehende Verarbeitungstechnologien ohne Prozessänderungen möglich
- Übliches Handling, z.B. durch Herstell- und Anwendbarkeit in Form von Masterbatches
- Niedrige Additivkonzentrationen (< 0,1%) genügen, um hohe biozide, vorzugsweise antibakterielle Wirksamkeiten zu realisieren
- Antibakterielle Wirkung in Langzeitversuchen und nach 50 Wäschen unter Industriebedingungen experimentell nachgewiesen
- mit diesen Komplexen additivierte Polyolefine und Polyester sind biokompatibel, weisen keine Zytotoxizität auf, d.h. sie bestehen Tests nach DIN EN ISO 10993-5, -4, -10 und besitzen vor allem hautpflegende Eigenschaften.

Polyester und Polyolefin zählen im Bereich der technischen Thermoplaste zu einer bedeutenden Gruppe polymerer Werkstoffe mit überwiegend hydrophoben Eigenschaften. Als wichtiger Konstruktionswerkstoff werden Polyester und Polyolefin vor allem als Polyester- und Polyolefinfaserstoffe für Textilien, Vliesstoffe und Mikrofasern eingesetzt, werden aber auch zu Folien oder PET-Flaschen verarbeitet.

Die vorliegende Erfindung betrifft durch Zinkionen funktionalisierte Polyester- und Polyolefinzusammensetzungen.

Die antibakterielle Ausrüstung von Polyester- und Polyolefin-Formmassen durch die Zugabe von Zinkpartikeln, meist in Form von Zinkoxid, ist bekannt. Der Nachteil von Zinkoxidpartikeln liegt darin, dass hier die Partikelgröße immer eine wichtige Rolle spielt, insbesondere bei der Verspinnung der Formmassen zu Fasern muss diese begrenzt sein. Zudem muss das Zink erst in eine wirksame lonenform umgewandelt werden, das heißt, das Zinkoxid muss erst gelöst werden und die Formkörper neigen oft zu Vergilbungen. Man kann diese Nachteile überwinden, indem man die Zinkoxidpartikel nur auf der Oberfläche in Beschichtungen und in Kombination mit Titandioxidpartikeln verwendet, wie zum Beispiel in der JP 2011-111704 offenbart.

Dieser Erfindung lag deshalb die Aufgabe zugrunde, die Polyester- und Polyolefinformmassen vor der Extrusion in-situ mit Zinkionen molekular auszurüsten, eine direkte Verarbeitung der Formmassen aus der Schmelze auch bei hohen Temperaturen zu ermöglichen und eine langfristige antibakterielle Wirkung zu gewährleisten. Der sonst übliche zusätzliche Verfahrensschritt der nachträglichen Oberflächenbeschichtung sollte vermieden werden, auch im Innern eines Formkörpers vorhandene Zinkionen sollten an die Oberfläche diffundieren und wirksam werden können.

Diese Aufgabe wird durch molekular mit Zinkionen ausgerüstete Polyester- und Polyolefinzusammensetzungen nach Anspruch 1 gelöst.

Die Aufgabe wurde gelöst durch eine Komplexierung von Zinksalzen mit Carbonsäure-(2-Oxazolin)-Derivaten, die thermodynamisch sehr stabile ZinksalzKomplexe bilden. Die Organoligand-Komplexe führen dazu, dass das Zink bereits in der wirksamen Form als Zinkion durch die Liganden stabilisiert wird, als solches in der Formmasse molekular vorliegt und eine mit Zinkionen homogen dotierte Ausrüstung möglich wird.

Die oben beschriebene Aufgabe wird nun dadurch gelöst, dass mindestens ein Polyester oder Polyolefin und mindestens ein Zinksalz-Organoligand-Komplex in der Polyester- und Polyolefinzusammensetzung vorliegt. Der mindestens eine Polyester ist bevorzugt ein Polyethylenterephthalat (PET), ein Polybutylenterephthalat (PBT) oder ein Polytrimethylen-terephthalat (PTT). Das mindestens eine Polyolefin ist bevorzugt ein Polyethylen oder Polypropylen.

Für die Funktionalisierung von Polyestern, insbesondere Polyethylenterephthalat oder Polybutylenterephthalat mit Zinksalz-Organoligand-Komplexen muss streng darauf geachtet werden, dass Komplexe unter Feuchteausschluss hergestellt und in wasserfreier Formulierung verwendet werden. Diese Aufgabe wird dadurch gelöst, dass bei der Herstellung der Zinksalz-Organoligand-Komplexe das Kristallwasser in situ entfernt wird.

Erfindungsgemäß können neben diesen Polymeren auch Copolyester und Copolyolefine, Block-Copolyester und Block-Copolyolefine und Polyester- oder Polyolefin-Blends verwendet werden.

Die erfindungsgemäßen komplexierten Zinksalzverbindungen sind stets durch eine Kombination aus einem Zinksalz mit einem Organoliganden aufgebaut. Das Zinksalz kann ein beliebiges Zinksalz sein, das aus einem Metallkation und einem anorganischen oder organischen Anion besteht. Beispiele besonders geeigneter Zinksalze sind Zink(II)-chlorid (ZnCl₂), Zink(II)-jodid (ZnJ₂), Zinksulfat (ZnSO₄) und Zink(II)-acetat (Zn(CH₃COO)₂). Die als Organoligand in den Metallsalz-Organoligand-Komplexen enthaltenen wirksamen Substanzen sind vorzugsweise 2-Oxazolinderivate organischer Carbonsäuren. Unsubstituierte 2-Oxazoline (auch bezeichnet als 4,5-Dihydrooxazole) sind heterozyklische 5-Ringverbindungen, die jeweils ein Sauerstoffatom, ein Stickstoffatom und eine Doppelbindung im Ring enthalten. Die erfindungsgemäß als Organoliganden verwendeten 2-Oxazoline enthalten die Heteroatome Sauerstoff in 1-Stellung, den Stickstoff in 3-Stellung, die Doppelbindung im heterozyklischen Ring befindet sich zwischen dem Kohlenstoffatom 2 und dem Stickstoffatom.

Erfindungsgemäß werden 2-Oxazoline als Derivate organischer Carbonsäuren verwendet. Die organische Carbonsäure kann irgendeine Carbonsäure sein, die aliphatischer, aromatischer oder aliphatisch-aromatischer Natur ist und monofunktionell, difunktionell oder polyfunktionell hinsichtlich der Zahl vorhandener Carbonsäuregruppen aufgebaut ist.

Beispiele geeigneter Carbonsäuren, aus denen 2-Oxazolinderivate erfindungsgemäß als Ligandensysteme zur Komplexierung von in den Zinksalzen enthaltenen Zinkkationen gewonnen werden können, sind die monofunktionellen Carbonsäuren Palmitinsäure [Chemical Abstracts Registration No. 57-10-3], Stearinsäure [57-11-4], Behensäure [112-85-6], Laurinsäure [143-07-7], Erucasäure [112-86-7], weiterhin die difunktionellen Carbonsäuren Oxalsäure [144-62-7], Adipinsäure [124-04-9],2-Bromisophthalsäure [22433-91-6], 4-Bromisophthalsäure [6939-93-1], 5-Bromisophthalsäure [23351-91-9], Isophthalsäure [121-91-5], Terephthalsäure [100-21-0], 2-Bromterephthalsäure [586-35-6] sowie die trifunktionelle Carbonsäure Trimesinsäure [554-95-0]. Es zeigte sich, dass insbesondere die monofunktionellen Carbonsäuren zur Bildung der 2-Oxazolinderivate als Ligandensystem geeignet sind für die Herstellung antibakteriell ausgerüsteter Polyester- und Polyolefinfasern, während die di- und trifunktionellen Carbonsäuren zu Komplikationen an den feinen Spinndüsen führen und deshalb besonders geeignet sind zur Herstellung von Polyester- oder Polyolefinfolien und Polyester- oder Polyolefin-Spritzgussartikeln. Überraschend wurde auch gefunden, dass mit Isophthalsäure [121-91-5], Terephthalsäure [100-21-0] und 2-Bromterephthalsäure [586-35-6] gebildete 2-Oxazolinderivate für die Eigenschaftsmodifizierung von Polyamiden sehr gut, für die Generierung von antibakteriellen Eigenschaften von Polyestern und Polyolefinen wesentlich weniger gut geeignet sind.

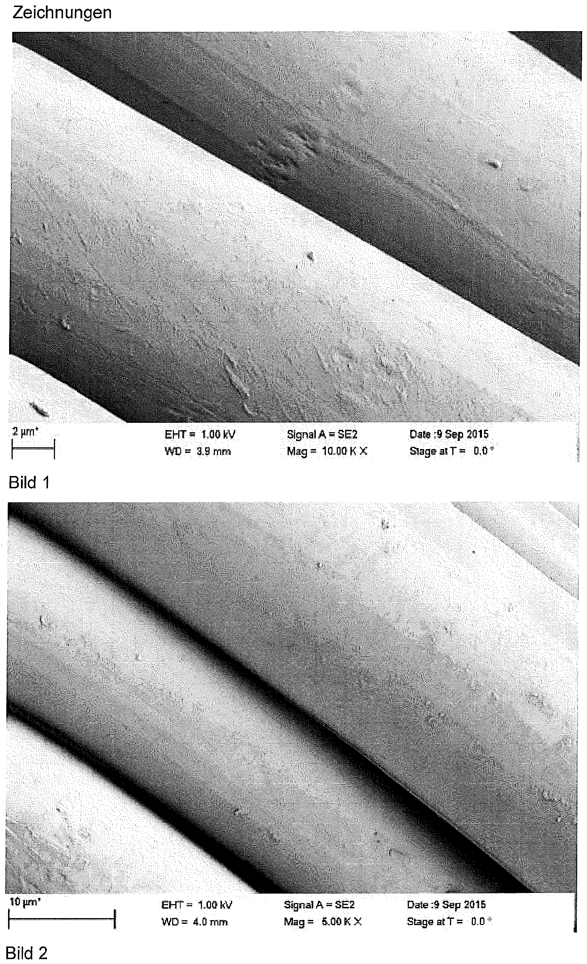

Die Zinkanteile sind in den erfindungsgemäßen Polyester- und Polyolefinzusammensetzungen durch ihre molekulare Gestalt als Organokomplex-Formulierungen homogen verteilt. In den REM-Aufnahmen Bild 1 (PET-Faser ohne Zink-Organoligand) und Bild 2 (PET-Faser mit 0,2 Masse-% Zinkiodid-Behensäure-2-Oxazolin-Komplex (entsprechend 250 ppm Zink in der Polyesterzusammensetzung)) sind vergleichsweise die Probenoberflächen von Fasern gezeigt. Es ist gut zu erkennen, dass die Zinkanteile der Probe 2 keine größeren Agglomerationen oder Partikel auf der Faseroberfläche gebildet haben. Diese homogene Zinkverteilung wird insbesondere durch die organophile Struktur der Liganden in den Organokomplexen hervorgerufen.

Der Verarbeitungsprozess der Polymerformmassen wird durch den Zusatz der Zinksalz-Organoligand-Komplexverbindungen in keiner Weise beeinträchtigt. Die mit den Zinkkomplexen additivierten Polyester- oder Polyolefinformmassen können aus Schmelzen wie üblich zu Formteilen durch Spritzgießen, Tiefziehen, Blasformen oder zu Monofilen oder Filamenten durch Schmelzspinn-Monofil- oder Multifilamentverarbeitungsprozesse verformt werden.

Für die Funktionalisierung von Polyolefinformmassen hat sich als vorteilhaft erwiesen, Zinksalz-Organoligand-Komplexverbindungen mit einem hohen Anteil an organischer Substanz, die durch die Struktur und die Stöchiometrie in der Gesamtformulierung der verwendeten Organoliganden gegeben ist, zu verwenden. Diese Aufgabe wird dadurch gelöst, dass bei der Herstellung der Zinksalz-Organoligand-Komplexverbindungen ein stöchiometrisches Verhältnis von Organoligand zu Zinkverbindung von vorzugsweise 2 mol Ligand : 1 mol Zinksalz eingehalten wird. Gute Verarbeitungseigenschaften, Kompatibilitäten und dauerhafte Wirkungen werden erreicht, wenn Zinksalz-Organoligand-Komplexverbindungen in Polyolefinformmassen eingesetzt werden, in denen ein elementares Kohlenstoff zu Zink-Verhältnis von 4,5:1 bis 10:1 (mol C : mol Zn), bevorzugt 8:1 bis 10:1, eingehalten wird.

Die Modifizierung der Polyester- oder Polyolefin-formmassen mit den komplexierten Zinksalzverbindungen führt zu keinen chemischen Veränderungen der Formmassen während der Schmelzeverarbeitung, wenn besondere Bedingungen des Feuchteausschlusses bei der gemeinsamen Verarbeitung der Zinksalz-Organoligand-Komplexverbindungen mit Polyestern beachtet werden. Die Viskositäten werden nicht verändert, die eingesetzten Zinkionen zeigen keine katalytische Wirkung in Bezug auf Polymerabbaureaktionen. Zudem ist eine Mattierung oder Erhöhung des Weißgrades durch Zusatz von Titandioxid nicht notwendig, die Produkte bleiben reinweiß, die Anfärbbarkeit wird nicht verändert.

Als besondere Ausführungsform der Erfindung erweist sich die universelle Verarbeitbarkeit der zinkhaltigen Formmassen aus der Schmelze zu Faserprodukten nach üblichen Schmelzspinntechnologien. Die antibakteriellen Polyester- und Polyolefin-Formmassen können zu Endlosfilamenten (Multifilen), Stapelfasern oder Monofilen ausgesponnen und zum Aufbau verschiedener Faserprodukte verwendet werden. Dabei ist es möglich, ein Faserprodukt vollständig aus der zinkhaltigen Faserart oder nur unter Zuhilfenahme anteiliger Einarbeitung derselben in Mischungen mit reinen Polymerfaserprodukten herzustellen. Auf diese Weise können verschiedene Produkte mit unterschiedlichen antibakteriellen Wirkungsstufen erzeugt werden.

Neben den textilen Anwendungen sind auch andere Anwendungsbereiche für Kunststoffprodukte aus Polyestern oder Polyolefinen mit permanenten antibakteriellen Eigenschaften denkbar, zum Beispiel Tür- und Handgriffe, in Kunststoffen im Sanitärbereich, in Kunststoffen in der Möbelindustrie, wie Büro- oder Krankenhausmöbel oder auch zur antibakteriellen Ausrüstung von Produkten der Medizintechnik. Alle diese Produkte zeichnen sich durch permanente antibakterielle Eigenschaften bei nur geringen Konzentrationen an Zinkionen aus, hervorgerufen durch die Komplexierung der Zinkionen und die molekulare Verteilung der Zinkionen.

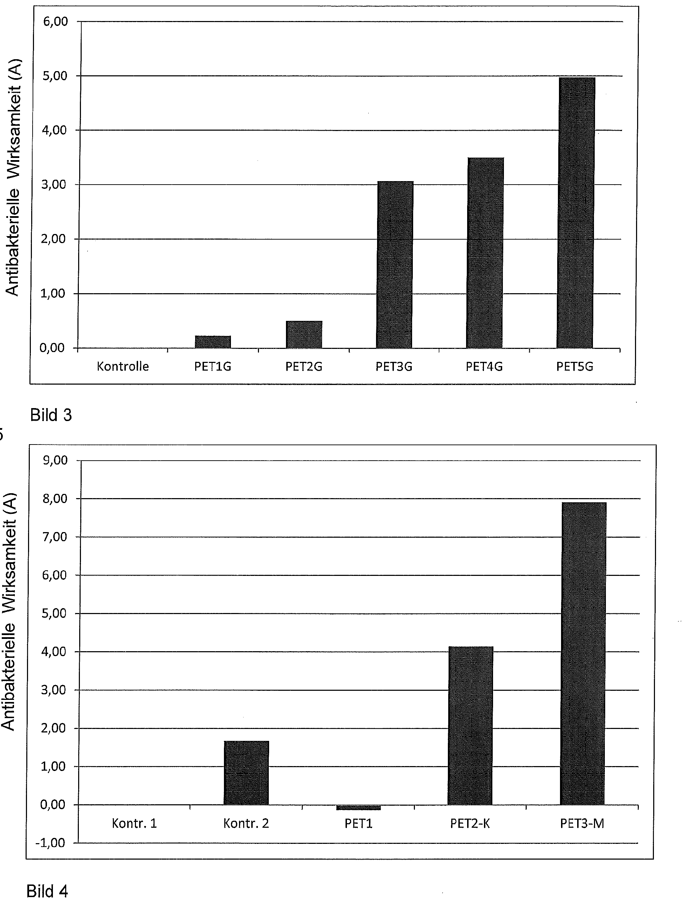

Die Menge an Zink, welche in Form der Zinksalz-2-Oxazolin-Komplexverbindungen zur wirksamen antibakteriellen Ausrüstung in die Polyester- oder Polyolefinformmassen eingebracht wird, ist in weiten Grenzen frei wählbar, solange die mechanischen Eigenschaften der Formmassen nicht negativ beeinflusst werden. Durch die organische Kapselung der Zinksalze durch die Organoliganden wird das Zink direkt in ionischer Form und molekular bereitgestellt. Das hat den Vorteil, dass die Zink-Dotierung deutlich unter den Mengenanteilen von häufig verwendeten partikulären Zinkoxiden liegt und auch die Zinkionen, die im Innern eines Formkörpers liegen, antibakteriell wirksam werden können. Üblicherweise liegt der Zinkanteil zwischen 50 und 500 ppm bezogen auf die Gesamtformulierung in Abhängigkeit von der geforderten antibakteriellen Wirksamkeit. Hervorragende Wirkungen wurden schon bei Zinkanteilen von 150 bis 300 ppm Zink bezogen auf die Gesamtformulierung nachgewiesen. In Fasern liegt der bevorzugte Konzentrationsbereich von Zinkanteilen zwischen 100 und 250 ppm Zink (Bild 3). Dabei ist es egal, ob die Faser als multifiles Endlosfilament, Monofil, BikomponentenFaser oder Stapelfaser oder ein Gewebe daraus hergestellt wurde (Tabelle 1, Bild 3).

Bild 3 zeigt Möglichkeiten der Einstellung der antibakteriellen Wirksamkeiten von Polyestergeweben durch die Konzentration der Zinkanteile in den verarbeiteten Fasern

**Tabelle 1: Antibakterielle Wirksamkeit von PET-Geweben mit verschiedenen Zinkanteilen, Prüfung nach DIN 20743; A>2: signifikante Wirkung; A>3: starke Wirkung**

| Gewebeprobe | Zink (ppm) | c_{B} [KBE/ml] | | Igc_{B} | | F | G | A | Stabw |
|---|---|---|---|---|---|---|---|---|---|
| | | 0h | 24h | 0h | 24h | log₂₄ₕ-log₀ₕ | log₂₄ₕ-log₀ₕ | (F - G) | |
| Kontrolle | 0 | 4,37E+04 | 6,61 E+07 | 4,6 | 7,8 | 3,18 | - | 0,00 | 0,08 |
| PET1G | 0 | - | 3,93E+07 | - | 7,6 | - | 2,96 | 0,22 | 0,06 |
| PET2G | 63 | - | 2,09E+07 | - | 7,3 | - | 2,68 | 0,50 | 0,08 |
| PET3G | 129 | - | 6,27E+05 | - | 4,7 | - | 0,11 | 3,07 | 2,12 |
| PET4G | 276 | - | 1,41E+05 | - | 4,3 | - | -0,32 | 3,50 | 1,74 |
| PET5G | 385 | - | 9,33E+02 | - | 2,8 | - | -1,79 | 4,97 | 0,47 |

Eine weitere erfindungsgemäße Ausführungsform ist die Möglichkeit, die zinkhaltigen Anteile in einer Faser mit vorbestimmten Anteilen gezielt und geometrisch definiert über den Faserquerschnitt verteilen zu können. Dabei bedient man sich einer speziellen Bi-Komponenten-Spinntechnologie, bei der gleichzeitig jeweils eine Zink-haltige und eine Zink-freie Polymerformmasse zu einer Bi-Komponenten-Spinndüse gefördert werden und daraus ein Faserprodukt abgezogen wird. Es können verschiedene Bi-Komponentfasern hergestellt werden, so z.B. mit "Kern-Mantel"-Struktur, vom Typ "Side-by-Side", "Isle-In-The-Sea" oder "Segmented Pie". Verwendet man eine Zink-haltige Polyester- oder Polyolefinformmasse in einem Spinnprozess zur Herstellung faserförmiger Produkte mit Kern-Mantel-Struktur, erhält man durch Dosierung der Zinkprodukte mit dem Mantelpolymeren beispielsweise Fasern, in denen an der Faseroberfläche Zinkdomänen angereichert sind, die schnell im späteren Gebrauch zu einer spontanen und wirksamen antibakteriellen Funktionalität führen. Speist man dagegen eine Zink-haltige Polymerformmasse in das Kernpolymer der Bi-Komponent-Faser ein, ist die antibakterielle Wirkung verzögert und besitzt vorteilhafterweise eine permanente Langzeitwirkung mit Depotfunktion. Dieser Effekt lässt sich an so hergestellten Kern/Mantelfasern sehr gut erklären, die mit jeweils gleicher Zinkkonzentration hergestellt wurden. Es wurde beobachtet, dass bei Kern/Mantelfasern, bei denen die Zinkdotierung in der Polymerformmasse des Mantels erfolgte, sehr schnell eine größere Menge an Zinkionen wirksam wird und die antibakterielle Wirkung somit höher ausfällt als in Fasern, in denen die Zinkdotierung nur in der Polyester- oder Polyolefin-Formmasse des Kerns vorgenommen wurde. Auch die Zinkionen aus der Kernfaser werden wirksam, können also an die Faseroberfläche des Mantels migrieren. Gleichzeitig ist die Wirkung der Zinkionen aus der Kernfaser hinsichtlich der antibakteriellen Ausrüstung verzögert und erfolgt über einen längeren Zeitraum. Auf diese Weise können vorteilhaft durch die gezielt eingebrachte Verteilungsstruktur der Zinkanteile in eine Polymerformmasse verschiedene Wirkungsstärken der antibakteriellen Ausrüstung eingestellt und auf die Anwendung der Formmassen ausgerichtet werden, wie am Beispiel von Kern-Mantel-Fasern gezeigt werden kann. Bild 4 zeigt die abgestufte Einstellung der antibakteriellen Wirksamkeit von Bi-Komponent-Polyester- und Polyolefinfasern mit Kern-Mantel-Struktur durch Verteilung der Zinkanteile im Kern (PET2-K) oder Mantel (PET3-M) der Faser.

**Tabelle 2: Antibakterielle Wirksamkeit von PET-Bi-Komponentfasern Typ Kern/Mantel mit gleichen Zinkanteilen im Kern (PET2-K) bzw. Mantel (PET3-M), Prüfung nach DIN 20743; A>3,3: signifikante Wirkung; A>7,9: biozide Wirkung**

| Bi-Ko-Faser | Zink (ppm) | c_{B} [KBE/ml] | | lgc_{B} | | F | G | A | Stabw |
|---|---|---|---|---|---|---|---|---|---|
| | | 0h | 24h | 0h | 24h | log₂₄ₕ-log₀ₕ | log₂₄ₕ-log₀ₕ | (F - G) | |
| Kontr. 1 | 0 | 5,20E+04 | 8,20E+07 | 4,7 | 7,91 | 3,25 | 3,25 | 0,00 | 0,10 |
| Kontr. 2 | Genta. | | 2,40E+06 | | 6,2 | | 1,59 | 1,67 | 0,42 |
| PET1 | 0 | - | 1,10E+08 | - | 8,03 | - | 3,38 | -0,13 | 0,09 |
| PET2-K | 340 | - | 7,80E+03 | - | 3,77 | - | -0,88 | 4,14 | 0,43 |
| PET3-M | 340 | - | 0,00E+00 | - | 0,00 | - | -4,65 | 7,91 | 0,00 |

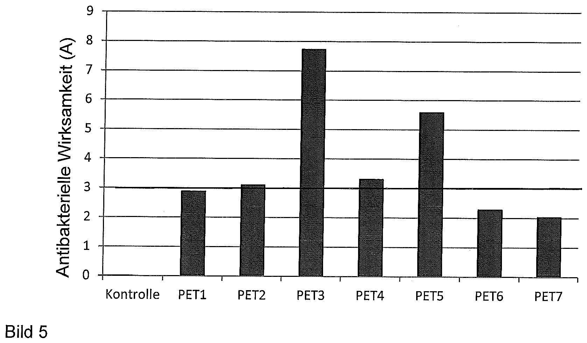

Die erfindungsgemäßen Polyester- und Polyolefin-Zusammensetzungen sind weiterhin hervorragend geeignet im Hinblick auf die Einstellung weiterer zusätzlicher Materialeigenschaften. Diese variable Modifizierungsmöglichkeit von Polyester- und Polyolefinformmassen ergibt sich insbesondere dadurch, dass verschiedene Zinksalze mit unterschiedlichen Anionen stabile Metallsalz-Organoligand-Komplexverbindungen bilden. Sehr vorteilhaft können dadurch die üblicherweise in Formmassen eingebrachten wirksamen Zinkanteile durch Verarbeitung unterschiedlicher Zinksalz-Komplex-Verbindungen erwirkt werden, je nachdem, welches Zinksalz mit welchem Organoliganden für die Komplexbildung benutzt wurde. Auf diese Weise ist es einfach zu realisieren, dass möglicherweise unerwünschte Anionen in der Formmasse vermieden werden können oder die antibakterielle Wirksamkeit der Formmassen abgeschwächt bzw. verstärkt werden kann, siehe Tabelle 3, Bild 5. So kann durch einfache Wahl der Zinkverbindung ein antibakterielles Wirkungsspektrum zwischen bakteriostatisch bis biozid bereits im Herstellungsprozess der Formgebung der Formmassen eingestellt werden. Auch andere antibakteriell wirksame Metallsalze, wie die Salze von Silber oder Kupfer können durch entsprechende Organoliganden komplexiert und einzeln oder in Mischungen mit Zink eingesetzt werden und können so zur Verstärkung der Wirkung beitragen.

**Tabelle 3: Antibakterielle Wirksamkeit von PET-Fasern mit verschiedenen Zinkzusätzen und -konzentrationen, Prüfung nach DIN 20743; A>2: signifikante Wirkung; A>3: starke Wirkung**

| Faserprobe | Zinksalz | Zn (ppm) | c_{B} [KBE/ml] | | lgc_{B} | | F | G | A | Stabw A |
|---|---|---|---|---|---|---|---|---|---|---|
| | | | 0h | 24h | 0h | 24h | log₂₄ₕ-log₀ₕ | log₂₄ₕ-log₀ₕ | (F - G) | |
| Kontrolle | - | 0 | 5,53E+04 | 5,40E+07 | 4,74 | 7,73 | 2,99 | 2,99 | 0,00 | 0,06 |
| PET 1 | - | 0 | | 7,50E+04 | - | 4,85 | - | 0,11 | 0,88 | 0,17 |
| PET 2 | ZnJ2 | 50 | | 5,10E+04 | - | 4,63 | - | -0,11 | 3,10 | 0,35 |
| PET 3 | ZnJ2 | 100 | | 0,00E+00 | - | 0,00 | - | -4,74 | 7,73 | 0,00 |
| PET 4 | ZnSO4 | 50 | | 4,80E+04 | - | 4,42 | - | -0,32 | 3,31 | 0,58 |
| PET 5 | ZnS04 | 100 | | 3,00E+03 | - | 2,14 | - | -2,60 | 5,59 | 1,99 |
| PET 6 | Zn(Ac)2 | 50 | | 3,00E+05 | - | 5,44 | - | 0,70 | 2,29 | 0,26 |
| PET 7 | Zn(Ac)2 | 100 | 7,50E+04 | 5,00E+04 | - | 5,69 | - | 0,95 | 2,04 | 0,11 |

Die mittels der erfindungsgemäßen komplexierten Zinksalzverbindungen in die Polymerformmassen eingebrachten Zinkanteile sind exktraktions- und emissionsbeständig. Bei Einkomponenten-Polyester- oder Polyolefinfasern, welche mit unterschiedlichen komplexierten Zinksalzverbindungen dotiert worden waren, konnte nahezu der ursprünglich eingebrachte Zinkgehalt auch nach bis zu 50 standardisierten Waschvorgängen festgestellt werden (Tabelle 4). Zudem wurde nach diesen Waschprozessen noch gute antibakterielle Wirkungen nachgewiesen.

**Tabelle 4: Beständigkeit der Zinkdotierung gegenüber wässriger Extraktion (Waschverfahren für Fasern nach DIN EN ISO 105-C08/C09:2003)**

| Multifilfaser | Im Komplex Verwendetes Zinksalz | Zinkgehalt (ppm) in der Faser | | |
|---|---|---|---|---|
| | | Ausgangswert | Nach 25 Wäschen | Nach 50 Wäschen |
| PET 1 | ZnJ₂ | 63 ± 6 | 57 ± 6 | 54 ± 6 |
| PET 2 | ZnSO₄ | 24 ± 3 | 19 ± 2 | 19 ± 2 |
| PET 3 | ZnJ₂ | 18 ± 2 | 14 ± 2 | 14 ± 2 |
| PET 4 | ZnJ₂ | 126 ± 12 | 121 ± 12 | 122 ± 12 |

Die aus der Schmelzeverarbeitung der erfindungsgemäßen Polyester- oder Polyolefinzusammensetzungen zu Filamenten, Monofilen oder Stapelfasern resultierenden Faserprodukte sind biokompatibel. Spezielle Untersuchungen nach DIN 10993-1 bis 10 haben gezeigt, dass von den Fasern keine Sensibilisierungsreaktionen ausgehen, diese kein hämolytisches Potential und auch keine zytotoxischen Eigenschaften besitzen.

Die erfindungsgemäßen Polyester- oder Polyolefin-Zusammensetzungen können ohne Einschränkungen mit weiteren üblichen Additiven, Füllstoffen, Verarbeitungshilfsmitteln, Farbpigmenten oder Gleitmitteln versehen und verarbeitet werden, ohne dass der Funktionalisierungseffekt beeinträchtigt wird. Durch die beschriebenen Vorteile eignen sich die Polyester- bzw. Polyolefin-Zusammensetzungen besonders zur Herstellung von Form- und Bauteilen, Platten und Folien. Einsatzgebiete sind hier insbesondere im Medizintechnikbereich sowie zur Verpackung von Lebensmitteln denkbar. Die Herstellung funktionalisierter Fasern oder Filamenten ist ebenso hervorragend möglich. Die Anwendungen liegen hier besonders im Bereich technischer Textilien oder antibakteriell ausgerüsteter textiler Fasern für Bekleidung mit geruchshemmenden Eigenschaften oder für medizinische Anwendungen oder auch im Bereich der Hygieneartikel.

Die Herstellung der Polymerzusammensetzungen erfolgt üblicherweise durch Mischen mindestens eines Polyesters oder Polyolefins mit mindestens einem Metallsalz-Organoligand-Komplex. Die Vermischung der Komponenten kann in gebräuchlichen Mischeinrichtungen erfolgen. Dabei wird Polyester oder Polyolefin in Chipsform, Plättchenform oder als Granulat mit einem pulverförmigen Organokomplex vorgemischt, die Mischung in einem geeigneten Apparat gemeinsam aufgeschmolzen und in der Schmelze durch Einwirkung von Scherkräften homogenisiert. Bevorzugt wird allerdings so verfahren, dass eine Vorabmischung eines Metallsalz-Organoligand-Metallkomplexes in Form eines Masterbatches oder Vorkonzentrates hergestellt und dieses später dem reinen Polyester- und Polyolefin bei der Herstellung des Halbzeuges oder Endproduktes zugemischt wird, gegebenenfalls mittels einer Seitenstromdosierung an einer Extrusionsmaschine. Der Anteil des Metallsalz-Organoligand-Komplexes in einem Masterbatch richtet sich nach der Verträglichkeit mit dem Matrixmaterial, dem beabsichtigten Funktionalisierungsgrad und der gewünschten Endkonzentration im Compound. Es ist möglich, Vorkonzentrate bis zu einer Konzentration von 20 Masseprozenten an Metallsalz-Organoligand-Komplex herzustellen. Eine bevorzugte Metallionenkonzentration in einer universell einsetzbaren Masterbatchformulierung beträgt 10.000 ppm Zinkgehalt.

Werden Zinkionen-haltige Metallsalz-Organoligand-Komplexverbindungen zur Herstellung von Wirkstoffkonzentraten in Form von Masterbatches mit Zinkionenkonzentrationen von beispielsweise 10.000 ppm Zink (entspricht 1 Masseprozent Zink) für die Funktionalisierung von Polyestern hergestellt, muss streng darauf geachtet werden, dass wasserfreie Zinksalz-Organoligand-Komplexverbindungen im Extrusionsprozess verwendet werden. Polyethylenterephthalat oder Polybutylenterephthalat können üblicherweise nur mit Restfeuchtegehalten von weniger als 0,001 Masse-% Feuchte (10 ppm) ohne Polymerabbau zu Fasern versponnen werden. Es wurde gefunden, dass beispielsweise die Zink-Organoligand-Komplexverbindung, die durch Verwendung von Zinksulfat-Heptahydrat hergestellt und mit Polybutylenterephthalat zu einem Masterbatch durch Schmelzeextrusion verarbeitet wurde, zu erheblichen Polymerabbauvorgängen führt. Dies kann auf das eingelagerte Kristallwasser, welches in dem Zinksalz-Heptahydrat zu einem Anteil von 37 Masseprozent in seinem Salz vorliegt und bei einer Zumischung 14 Masseprozent zur Erzielung einer Zinkionenkonzentration von 10.000 ppm ca. 500 ppm Feuchte in die Formmasse einbringt, zurückgeführt werden, was schließlich den erheblichem Abbau der Polyestermatrix verursacht. Wird dagegen wasserfreies oder Zinksulfat-Monohydrat zur Formulierung der Zinksalz-Organoligand-Komplexverbindung verwendet, tritt das Problem des hydrolytischen Polymerabbaues im Extrusionsprozess nicht auf. Die experimentellen Versuchsdaten zeigt die Tabelle 5.

**Tabelle 5: Vergleich der Intrinsic Viscosity von PET-Masterbatches nach Formulierungen mit einem Zinksalz-Organoligand-Komplex, bestehend aus Laurinsäure-Organoligand Zinksulfat-Heptahydrat (PET 2) bzw. Zinksulfat-Monohydrat (PET 3) im Vergleich zur Polyestermatrix ohne Zusatz (PET)**

| Masterbatch 10.000 ppm Zink | Im Komplex verwendetes Zinksalz | Zinkgehalt Masterbatch (ppm) | Intrinsic viscosity (IV) | |
|---|---|---|---|---|
| | | | 1. Wert | 2. Wert |
| PET | ohne | 0 | 0,63 | 0,62 |
| PET 2 | ZnSO₄ x 7 H₂O | 9887 ± 100 | 0,41 | 0,38 |
| PET 3 | ZnSO₄ x 1 H₂O | 9946 ± 100 | 0,60 | 0,61 |

Das mit dem Zinksulfat-Heptahydrat funktionalisierte Masterbatch PET 2 zeigte erheblichen Polymerabbau, das Material kann nicht stabil zur Herstellung von Polyester-Multifilamenten verwendet werden.
Die Aufgabe wurde nun dadurch gelöst, dass zur Synthese der Zinksalz-Organoligand-Komplexverbindung auf Basis von Zinksulfat das Heptahydrat in ethanolischer Lösung in situ entwässert und auf diese Weise in sein Monohydrat überführt und in dieser Form zur Bildung des Zinksulfat-Organoligand-Komplexes verwendet wird und keine zusätzliche Polymerschädigung mehr verursacht (PET 3).

Die bioaktiv in situ bzw. biocid in situ, vor allem antibakteriell in situ funktionalisierten Polyester- oder Polyolefinzusammensetzungen gemäß dieser Erfindung sowie die in bekannten Verfahren daraus hergestellten funktionalisierten Formkörper und Produkte bestehen aus aromatischen und/oder aliphatischen Polyestern oder Polyolefinen. Unter anderem - jedoch nicht darauf beschränkt - enthalten sie Polyethylenterephthalat (PET), Polytrimethylenterephthalat (PTT), Polybutylenterephthalat (PBT), Polyglycoliden (PGA), Polylactiden (PLA), Polyhydroxyalkanoaten (PHA, PHB), aromatische und/oder aliphatische Polyester- und Polyolefincopolymere sowie Polyester- und Polyolefin- und Copolyester- und Polyolefinmischungen und/oder thermoplastische Elastomere, unter anderem - jedoch nicht darauf beschränkt - solche auf Ester-Basis. Zudem können duromere Polyesterformmassen derart erfindungsgemäß funktionalisiert sein. In Folge eines dem Stand der Technik entsprechenden Verarbeitungs- und Herstellungsprozesses entstehen darauf basierende -jedoch nicht darauf beschränkt- Monofilamente, Multifilamente und/oder Fasern, auch Flockfasern sowie Produkte daraus. Es lassen sich darauf basierende Folien, Spritzgussteile sowie weitere Produkte herstellen oder die erfindungsgemäß bioaktiv in situ bzw. biocid in situ, vor allem antibakteriell in situ funktionalisierten Polymerzusammensetzungen finden in entsprechenden bekannten Applikationen Anwendung. Alle derart industriell hergestellten Produkte und Folgeprodukte oder industriell genutzte Applikationen sind jeweils gekennzeichnet durch inhärente langanhaltende Permanenz und inhärent hohe Effizienz der resultierenden bioaktiven bzw. biociden, vor allem der antibakteriellen Wirksamkeit. Die bioaktive Wirksamkeit kann eingestellt werden durch die Konzentration der Zinkionen in der Polymerzusammensetzung und durch die Konstruktion des Formkörpers. Durch die Höhe der Dotierung der Polymerformmassen mit den Zinkionen und bei Fasern und Filamenten durch die Herstellung von Bikomponentenfasern lassen sich somit die Wirkungsweise, die Abgaberate und die Dauer der Wirkung einstellen. So konnte zum Beispiel bei Kern/Mantelfasern immer eine antibakterielle Wirkung des Zinks nachgewiesen werden, unabhängig davon, ob der Zinksalz- 2 Oxazolin-Komplex sich im Mantel oder im Kern der Bikomponentenfaser befand. Erfolgt die Dotierung im Mantel erfolgt die Wirkstofffreisetzung schneller und in höheren Raten, bei einer Dotierung des Kerns wird die Wirkstofffreisetzung langsamer, dafür über einen längeren Zeitraum.

Die unter Einsatz erfindungsgemäß hergestellter bioaktiv in situ bzw. biocid in situ, vor allem antibakteriell in situ funktionalisierter Polyester- und Polyolefinzusammensetzungen gewonnenen Produkte und die entsprechenden Applikationen zeichnen sich zudem durch eine außergewöhnliche Umwelt- und Hautverträglichkeit sowie durch eine überaus geringe Zytotoxizität aus und die inhärente Bioaktivität, vor allem die inhärente antibakterielle Wirkung besteht dauerhaft, da Mikroorganismen aller Art keine Resistenzen ausbilden.

### Beispiel 1:

### Laurinsäure-(2-oxazolin)-Zn(II)-sulfat-Komplex (Zn(II)-12S)

Der Organoligand wurde aus Laurinsäure über einen Veresterungsschritt mit anschließender Aminolyse mit Ethanolamin und darauf folgender dehydratisierender Ringschlussreaktion unter Einwirkung von Katalysatoren auf folgende Weise hergestellt:
100 g Laurinsäure wurden mit 600 ml Methanol und 20 ml konz. Salzsäure gemischt und 3 Std. am Rückfluss gekocht. Die Hauptmenge Methanol wurde abdestilliert, nach dem Abkühlen wird die organische Phase abgetrennt. Der Rohester wurde drei Mal mit Eiswasser gewaschen, getrocknet und unter Vakuum fraktioniert. Das Produkt wurde unter einem Vakuum von 0,1 mbar bei 100 bis 120 °C abgenommen.

| | | | |
|---|---|---|---|
| Ausbeute: | 94 %, flüssig | VZ: | 262,5 (th. 261,7) |

Den Laurinsäure-(2-oxazolin)-Zn(II)-sulfat-Komplex wurde in einer Rührapparatur erhalten, indem zunächst 10 g Zinksulfat-Heptahydrat in Anwesenheit von Hydratwasser in 30 ml getrocknetem Ethanol gelöst und diese Mischung für 10 Minuten an mäßigem Sieden gehalten wurde. Auf diese Weise wurde das Heptahydrat durch Ligandenaustausch dehydratisiert, wobei sich das Monohydrat bildete. Der Dehydratisierungsvorgang ließ sich verfolgen, indem aus der ethanolischen Mischung die flüssige Phase entnommen und nach Zusatz von weißem Kupfersulfat auf Anwesenheit von Dehydratwasser geprüft wurde. Wird keine Blaufärbung mehr beobachtet, liegt Zinksulfat-Monohydrat vor. Dieses wurde abgesaugt und bei 75 °C getrocknet.

Zur Komplexbildung wurden 2,85 g Zinksulfat-Monohydrat in getrocknetem Ethanol vorgelegt und dazu eine Lösung von 5,0 g Laurinsäureligand in 50 ml trockenem Ethanol zugetropft. Unter Rühren bildete sich ein weißer Niederschlag, die Mischung wurde weitere 2 Tage bei Raumtemperatur nachgerührt. Durch Absaugen und Trocknen wurde der Komplex gewonnen.

Ausbeute: 5-6 g

| Elementaranalyse: | C | H | N | Zn |
|---|---|---|---|---|
| theor.: | 54,9 | 8,9 | 4,6 | 10,7 |
| gef.: | 52,9 | 8,6 | 4,0 | 11,0 |

Ligandenverhältnis: 2:1
Kohlenstoff-Zinkverhältnis: 4,8 : 1

Das folgende Beispiel war für die Funktionalisierung von Polyolefinen besonders geeignet.

### Beispiel 2:

### Behensäure-(2-oxazolin)-Zn(II)-acetat-Komplex (Zn(II)-22 Ac)

Der Organoligand aus Behensäure wurde analog zum Beispiel 1 hergestellt, indem 100 g Behensäure mit 800 ml Methanol und 45 ml konz. HCl gemischt und 3 Std. am Rückfluss unter N₂ gehalten wurde, und die Mischung dann noch heiß in ein Becherglas umgefüllt und unter Rühren langsam abgekühlt wurde. Dabei fiel der Rohester in granulierter Form an. Er wurde abfiltriert, in 150 ml Wasser gegeben und diese Mischung mit 5%-iger K₂CO₃-Lösung neutralisiert. Das feste Produkt wurde erneut abgesaugt und aus 200 ml Aceton umkristallisiert. Über Nacht im Kühlschrank kristallisierte der Ester als feiner Niederschlag aus, er wurde abgesaugt und bei 40 °C im Vakuum getrocknet.

| | | | |
|---|---|---|---|
| Ausbeute: | 96 % | Fₚ: | 52-54 °C |

Verseifungszahl: 154,7

Der Organoligand wurde hergestellt, indem 142 g Behensäuremethylester, 61 g Ethanolamin und 1,4 g Titan(IV)-butylat unter N₂-Atmosphäre langsam auf 140-175°C erhitzt wurden und dabei Methanol abdestilliert wurde. Danach wurde unter reduziertem Druck zunächst bei ca. 400 mbar, dann bei 20 mbar überschüssiges Ethanolamin entfernt, bis die Temperatur im Kühler sank. Anschließend wurde das Rohoxazolin im Vakuum destilliert. Unter einem Vakuum von 0,038 mbar wurde das Produkt in Fraktionen zwischen 205 und 245 °C Kopftemperatur abgenommen.

| | | | |
|---|---|---|---|
| Ausbeute: | 105 g (48 %) | Fₚ: | 57 °C |
| Reinheit: | 85 - 92 % | | |

Der Zinkacetat-Organoligand-Komplex wurde erhalten, indem der Organoligand aus Behensäure mit Zink(II)-Acetat umgesetzt wurde. In einer Rührapparatur wurden zunächst 10 g Zink(II)-Acetat-Dihydrat in Anwesenheit des Hydratwassers in 30 ml getrocknetem Ethanol gelöst. Diese Mischung wurde für 7 Minuten bei mäßigem Sieden gehalten. Dabei wurde das Dihydrat durch Ligandenaustausch dehydratisiert und das Monohydrat bildete sich. Der Dehydratisierungsvorgang ließ sich verfolgen, indem aus der ethanolischen Mischung die flüssige Phase entnommen und nach Zusatz von weißem Kupfersulfat auf Anwesenheit von Dehydratwasser geprüft wurde. Wurde keine Blaufärbung mehr beobachtet, liegt Zinksulfat-Monohydrat vor. Dieses wurde abgesaugt und bei 75 °C getrocknet.

Zur Komplexbildung wurden 1,8 g Zink(II)-acetat-Monohydrat in getrocknetem Ethanol vorgelegt und dazu eine Lösung aus 7,3 g Organoligand aus Behensäure in 100 ml trockenem Ethanol zugetropft. Es wurde weiter gerührt und nach 2 Tagen der Komplex als Niederschlag gewonnen, der anschließend absaugt und trocknet wurde.

Ausbeute: 4-5 g

| Elementaranalyse: | C | H | N | Zn |
|---|---|---|---|---|
| theor.: | 68,3 | 11,0 | 3,0 | 7,1 |
| gef.: | 68,1 | 11,3 | 3,0 | 6,4 |

Ligandenverhältnis: 2:1
Kohlenstoff-Zinkverhältnis: 9,7 : 1

### Beispiel 3:

In einem Doppelschneckenextruder Haake Rheomex® PTW16/25 p mit einer Drehzahl von 120 min⁻¹ wurde bei 270 °C ein Masterbatch aus dem Homopolymer-Polyester 4048 der Firma Invista und dem Zinksalz - Organoligand- Oxazolin-Komplex hergestellt.
Variante A- Zinkiodid-Laurinsäure -Komplex mit 1250 ppm Zn
Variante B- Zinksulfat-Behensäure -Komplex mit 1000 ppm Zn
Variante C- Zinkacetat-Behensäure -Komplex mit 1000 ppm Zn

### Beispiel 4:

In der Schmelzspinnanlage wurde das PET 4048 ohne Zusätze als Vergleichsprobe (PET1) beziehungsweise das entsprechende Masterbatch mit dem Polyestergranulat 4048 gemischt, so dass Spinnschmelzen mit den folgenden Zusammensetzungen entstanden:
PET2 - Masterbatch A, 50 ppm Zn²⁺
PET3 - Masterbatch A, 100 ppm Zn²⁺
PET4 - Masterbatch B, 50 ppm Zn²⁺
PET5 - Masterbatch B, 100 ppm Zn²⁺
PET6 - Masterbatch C, 50 ppm Zn²⁺
PET7 - Masterbatch C, 100 ppm Zn²⁺

Die Schmelzen wurden über Spinndüsenpakete mit einer 24-Loch Spinndüse zu Fasern der Feinheit 250 dtex versponnen und auf Spulen gespeichert.

### Beispiel 5:

Die Fäden der Spinnspulen wurden mit einem Reckverhältnis von 1:3 zu einem POY-Glattgarn verstreckt und die Fadenparameter bestimmt (siehe Tabelle 3a)

**Tabelle 6: Gegenüberstellung der Fadenparameter**

| Probe | Feinheit | Zugdehnung | Zugfestigkeit | E-Modul |
|---|---|---|---|---|
| | dtex | % | cN/tex | MPa |
| PET1 | 81 | 28 | 45 | 11287 |
| PET2 | 83 | 40 | 40 | 11050 |
| PET3 | 83 | 41 | 38 | 10855 |
| PET4 | 85 | 40 | 36 | 10364 |
| PET5 | 84 | 38 | 34 | 9998 |
| PET6 | 82 | 33 | 37 | 10594 |
| PET7 | 83 | 42 | 36 | 10200 |

Es zeigt sich dass die Zugdehnung im Vergleich zur Vergleichsprobe bei allen Proben erhöht war und bis auf 150 % der Vergleichsprobe steigt. Zugfestigkeit und E-Modul reduzieren sich bei allen Proben, bei Probe 5 sank die Zugfestigkeit auf 76% des Vergleichsprobenwerts, der E-Modul auf 89 %.

### Beispiel 6:

Die in den Versuchen 8 bis 10 hergestellten Fasern wurden hinsichtlich ihrer antibakteriellen Wirksamkeit getestet, siehe Ergebnisse in Tabelle 3. Es zeigte sich, dass die Zinkiodid-Laurinsäure-Komplexe mit 100 ppm Zn²⁺ die stärkste antibakterielle Wirkung aufwiesen, gefolgt von den Zinksulfat-Behensäure-Komplexen mit 100 ppm Zn²⁺. Auch die Zinkiodid-Laurinsäure- und Zinksulfat-Behensäure-Komplexe mit Zn²⁺-Anteilen von 50 ppm zeigten noch eine sehr starke antibakterielle Wirkung. Die Zinkacetat-Behensäure-Komplexe wiesen bei beiden Zinkionen-Konzentrationen eine immer noch signifikante antibakterielle Wirkung auf.

## Patentansprüche

1. Polyester- oder Polyolefin-Zusammensetzungen, **dadurch gekennzeichnet, dass** sie mit Zinksalz-2-Oxazolin-Komplexen dotiert und dadurch bioaktiv funktionalisiert sind, das Zink in ionischer Form vorliegt und die Zinkionen molekular in der Polymerzusammensetzung verteilt sind, wobei der Anteil der Zinkionen im Bereich von 20 ppm bis 10000 ppm, bezogen auf das Gesamtgewicht der Zusammensetzung, liegt.

2. Zusammensetzung gemäß Anspruch 1, **dadurch gekennzeichnet, dass** der Anteil an Zinkionen im Bereich von 50 bis 500 ppm liegt, jeweils bezogen auf das Gesamtgewicht der Zusammensetzung.

3. Zusammensetzung nach Anspruch 1, **dadurch gekennzeichnet, dass** die Zinkionen in Zinksalz-Komplexverbindungen enthalten sind, wobei das Zinksalz Zinkjodid, Zinkchlorid, Zinkacetat oder Zinksulfat ist.

4. Zusammensetzung nach Anspruch 3, **dadurch gekennzeichnet, dass** die Komplexverbindung ein 2-Oxazolinderivat aliphatischer oder aromatischer Carbonsäuren oder Dicarbonsäuren umfasst.

5. Zusammensetzung nach Anspruch 4, **dadurch gekennzeichnet, dass** die 2-Oxazolinderivate Derivate der Palmitinsäure, Stearinsäure, Behensäure, Laurinsäure, Erucasäure, Oxalsäure, Adipinsäure, 2-Bromisophthalsäure, 4-Bromisophthalsäure, 5-Bromisophthalsäure, Isophthalsäure, Terephthalsäure, 2-Bromterephthalsäure oder der Trimesinsäure sind.

6. Zusammensetzung nach Anspruch 1, **dadurch gekennzeichnet, dass** der Polyester ein aromatischer und/oder aliphatischer Polyester ist.

7. Formkörper in der Form von Monofilamenten, Multifilamenten und/oder Fasern, Flockfasern, Multikomponentenfasern, Folien oder Spritzgussteilen hergestellt aus der bioaktiv funktionalisierten Polymerzusammensetzung nach mindestens einem der Ansprüche 1 bis 6, die permanente antibakterielle Eigenschaften aufweisen.

8. Formkörper nach Anspruch 7, **dadurch gekennzeichnet, dass** die bioaktive Wirkung langanhaltend ist und nach 50 Waschgängen noch mindestens 90 % der Zinkionen vorhanden und wirksam sind.

9. Formkörper nach Anspruch 7 oder 8, **dadurch gekennzeichnet, dass** er als Kern-Mantelfaser ausgebildet ist und die Zinkionen-haltige Polyester- oder Polyolefin-Zusammensetzung entweder im Mantel oder im Kernpolymer enthalten ist oder in unterschiedlichen Konzentrationen in Kern und Mantel.

10. Bioaktiver Polymerformkörper nach Anspruch 7, **dadurch gekennzeichnet, dass** die Zinkionenkonzentration im Formkörper zwischen 50 und 500 ppm liegt.

11. Verfahren zur Herstellung der Formkörper nach einem oder mehreren der Ansprüche 7 bis 10, **dadurch gekennzeichnet, dass**
- wasserfreie Polyester oder Polyolefine mit wasserfreien Zinksalz-Organoligand-Komplexen vermischt werden, wobei Kristallwasser aus den Komplexen in situ entfernt wird,
- die Mischung in eine Schmelze überführt und
- die Schmelze zu einem Formkörper geformt wird.

## Claims

1. Polyester or polyolefin compositions, **characterized in that** they have been doped with zinc salt/2-oxazoline complexes and thereby bioactively functionalized, the zinc is present in ionic form and the zinc ions are molecularly distributed in the polymer composition, wherein the content of the zinc ions is in the range from 20 ppm to 10 000 ppm, based on the total weight of the composition.

2. Composition according to Claim 1, **characterized in that** the content of zinc ions is in the range from 50 to 500 ppm, each based on the total weight of the composition.

3. Composition according to Claim 1, **characterized in that** the zinc ions are present in zinc salt complexes, wherein the zinc salt is zinc iodide, zinc chloride, zinc acetate or zinc sulfate.

4. Composition according to Claim 3, **characterized in that** the complex compound comprises a 2-oxazoline derivative of aliphatic or aromatic carboxylic acids or dicarboxylic acids.

5. Composition according to Claim 4, **characterized in that** the 2-oxazoline derivatives are derivatives of palmitic acid, stearic acid, behenic acid, lauric acid, erucic acid, oxalic acid, adipic acid, 2-bromoisophthalic acid, 4-bromoisophthalic acid, 5-bromoisophthalic acid, isophthalic acid, terephthalic acid, 2-bromoterephthalic acid or trimesic acid.

6. Composition according to Claim 1, **characterized in that** the polyester is an aromatic and/or aliphatic polyester.

7. Moulded article in the form of monofilaments, multifilaments and/or fibres, floc fibres, multicomponent fibres, films or injection mouldings produced from the bioactively functionalized polymer composition according to at least one of Claims 1 to 6, which have permanent antibacterial properties.

8. Moulded article according to Claim 7, **characterized in that** the bioactive action is long-lasting and after 50 washings at least 90 % of the zinc ions are still present and active.

9. Moulded article according to Claim 7 or 8, **characterized in that** it is structured as a core/sheath fibre and the zinc ion-containing polyester or polyolefin composition is present either in the sheath or in the core polymer or in different concentrations in core and sheath.

10. Bioactive moulded polymer article according to Claim 7, **characterized in that** the zinc ion concentration in the moulded article lies between 50 and 500 ppm.

11. Method for producing the moulded articles according to one or more of Claims 7 to 10, **characterized in that**
- anhydrous polyesters or polyolefins are mixed with anhydrous zinc salt organoligand complexes, wherein water of crystallization is removed from the complexes in situ,
- the mixture is converted to a melt and
- the melt is shaped to a moulded article.

## Revendications

1. Compositions de polyester ou polyoléfiniques, **caractérisées en ce qu'**elles sont dopées à l'aide de complexes de sel de zinc-2-oxazoline et **en ce qu'**elles sont fonctionnalisées de manière bioactive, de sorte que le zinc est présent sous forme ionique et les ions zinc sont répartis de manière moléculaire dans la composition polymère, la quantité des ions zinc se situant dans la plage allant de 20 ppm à 10 000 ppm par rapport au poids total de la composition.

2. Composition selon la revendication 1, **caractérisée en ce que** la quantité d'ions zinc se situe dans la plage allant de 50 à 500 ppm, respectivement par rapport au poids total de la composition.

3. Composition selon la revendication 1, **caractérisée en ce que** les ions zinc sont contenus dans les composés complexes de sel de zinc, le sel de zinc étant de l'iodure de zinc, du chlorure de zinc, de l'acétate de zinc ou du sulfate de zinc.

4. Composition selon la revendication 3, **caractérisée en ce que** le composé complexe comprend un dérivé de 2-oxazoline d'acides carboxyliques ou d'acides dicarboxyliques aliphatiques ou aromatiques.

5. Composition selon la revendication 4, **caractérisée en ce que** les dérivés de 2-oxazoline sont des dérivés d'acides palmitiques, d'acides stéariques, d'acides béhéniques, d'acides lauriques, d'acides éruciques, d'acides oxaliques, d'acides adipiques, d'acides 2-bromoisophtaliques, d'acides 4-bromoisophtaliques, d'acides 5-bromoisophtaliques, d'acides isophtaliques, d'acides téréphtaliques, d'acides 2-bromotéréphtaliques ou d'acides trimésique.

6. Composition selon la revendication 1, **caractérisée en ce que** le polyester est un polyester aromatique et/ou aliphatique.

7. Corps moulé sous la forme de monofilaments, de multifilaments et/ou de fibres, de fibres à floquer, de fibres multicomposées, de feuilles ou de pièces moulées par injection fabriqué à partir de compositions polymères fonctionnalisées de manière bioactive selon au moins l'une quelconque des revendications 1 à 6, qui présentent des propriétés antibactériennes permanentes.

8. Corps moulé selon la revendication 7, **caractérisé en ce que** l'effet bioactif est prolongé et après 50 cycles de lavage encore au moins 90 % des ions zinc sont présents et efficaces.

9. Corps moulé selon la revendication 7 ou 8, **caractérisé en ce qu'**il est formé en tant que fibre noyau-gaine et **en ce que** la composition de polyester ou polyoléfinique contenant des ions zinc est contenue soit dans la gaine, soit dans le polymère possédant un noyau ou présente en différentes concentrations dans le noyau et dans la gaine.

10. Corps polymère moulé de manière bioactive selon la revendication 7, **caractérisé en ce que**
la concentration en ions zinc dans le corps moulé est comprise entre 50 et 500 ppm.

11. Procédé de fabrication d'un corps moulé selon l'une ou plusieurs quelconques des revendications 7 à 10, **caractérisé en ce que**
- des polyesters ou polyoléfines anhydres sont mélangés à des complexes sel de zinc-organoligand, l'eau de cristallisation étant éliminé *in situ* des complexes,
- le mélange étant transféré dans une masse fondue et
- la masse fondue étant façonnée en un corps moulé.
